# EUROPEAN PATENT SPECIFICATION

(11) **EP 3 629 946 B1**
(45) Date of publication and mention of the grant of the patent: **30.06.2021**
(21) Application number: 18730871.3
(22) Date of filing: 29.05.2018
(51) Int. Cl.: A61B 17/14

(54) **AN ULTRASONIC CUTTING DEVICE FOR OSTEOTOMY**
ULTRASCHALLSCHNEIDVORRICHTUNG FÜR OSTEOTOMIE
DISPOSITIF DE COUPE ULTRASONIQUE POUR OSTÉOTOMIE

(30) Priority: 31.05.2017 IT 201700059460
(43) Date of publication of application: 08.04.2020
(73) Proprietor: Mectron S.p.A., 16042 Carasco GE (IT)
(72) Inventor: STABILINI, Elisa, 16040 Leivi (Genova) (IT); CHIRICO, Andrea, 16039 Sestri Levante (Genova) (IT); CERISOLA, Nicol, 16043 Chiavari (Genova) (IT); BOZZINI, Giorgio, 16126 Genova (IT)
(74) Representative: Soldatini, Andrea
(86) International application number: PCT/IB2018/053791
(87) International publication number: WO 2018/220515

(56) References cited:
- EP-A1- 2 374 428
- US-A- 5 188 102
- US-A- 5 695 510
- US-A1- 2004 023 187

## Description

### Technical field of the invention

The present invention relates generally to the field of osteotomy cutting devices. More particularly, the invention relates to a minimally invasive blade cutting device for ultrasound osteotomy.

### Background

In the field of surgery, it is often necessary to cut bone tissues, for example, for the installation of prostheses or for the application of screws, nails and, more generally, support elements for the repair of damaged bones following traumas of various nature. Cutting of bone tissues is also necessary during spinal and cranial surgery.

Among the cutting devices for osteotomy, ultrasound devices are extremely common, which include a vibration generator with an ultrasonic frequency, typically of the order of a few tens of kHz, for example 20 kHz or higher, to which an appropriately shaped blade can be connected through a special attachment and an extension.

Known ultrasonic cutting devices comprise a flat blade which develops in a longitudinal direction and is shaped like a hacksaw or a chisel. In this case, the vibration generator is configured to generate longitudinal vibrations, parallel to the blade development direction, which favour the cutting action with reciprocating movement exerted manually by a surgeon.

The use of ultrasonic cutting devices is preferred over that of traditional, manual or motorized cutting devices, because the ultrasonic vibrations favour the penetration of the blade reducing the force required by a surgeon, which allows greater precision in making a cut.

Examples of ultrasonic cutting devices are described in patents EP1311197, US5261922, US5695510, US8343178 and in the international patent publication WO2006/059120 A1.

Ultrasonic cutting devices can be improved as regards the blade guide and penetration features, both considering the increasingly felt need for a high cutting precision, and the fact that the blade must generally cut through bone tissues of varying consistency and thickness without damaging the underlying organs and/or tissues, especially in the case of spinal and cranial surgery, in which damage to the dura mater should be minimized.

The need to provide ultrasonic cutting devices which allow obtaining high cutting precisions independently of the manual skill of a surgeon is also strongly felt.

### Summary of the invention

The technical problem posed and solved by the present invention is therefore that of providing an improved ultrasonic cutting device which allows overcoming the drawbacks mentioned above with reference to the prior art.

This problem is solved by an ultrasonic cutting device according to claim 1.

Preferred features of the present invention are defined by the dependent claims.

The ultrasonic cutting device for osteotomy according to the invention comprises a blade and an attachment member configured to be connected to a longitudinal vibration generator. The blade comprises a proximal portion connected to the attachment member and a distal portion which carries a plurality of cutting elements. Such cutting elements comprise a portion of arcuate shape obtained at the free end of the blade in the longitudinal direction and on which there is a continuous cutting profile. The cutting elements further comprise a plurality of teeth obtained on both sides of the blade symmetrically with respect to a longitudinal axis thereof. The teeth are aligned with each other along incident directions with respect to the longitudinal axis of the blade, so that the overall configuration of the distal portion is that of a wedge which widens from the arcuate portion in the proximal direction.

The main advantage provided by the invention is that of combining a continuous cutting profile with a pair of discontinuous cutting profiles respectively consisting of a plurality of teeth, in which each cutting profile has a specific function. In particular, the continuous cutting profile obtained at the free end in the longitudinal direction serves for guiding the device during the initial penetration step in the bone tissue, and offers a surgeon a good tactile feedback due to the continuous contact between the latter and the blade. The teeth arranged in alignment along directions which are incident with respect to the longitudinal axis of the blade allow instead to progressively widen the slit produced initially in the bone by the arcuate portion, without requiring a surgeon to move the blade sideways.

In other words, with respect to known ultrasonic cutting devices, the one according to the invention allows a surgeon to concentrate more on the precision of the cut and its depth, being able to rely on a blade geometry which autonomously provides for widening the produced slit, as the cutting device is advanced into the bone tissue.

According to an embodiment of the invention, the cutting profile of the arcuate portion is formed as a chamfer in the thickness direction of the blade, which simplifies manufacture in particular considering the dimensions and millimetric thicknesses of the blades of the ultrasonic cutting devices for osteotomy.

According to an embodiment of the invention, the ultrasonic cutting device comprises a plurality of indentations formed in the longitudinal direction in the vicinity of the arcuate portion. The function of the indentations is to improve the effect of frontal penetration of the device.

Further advantages, features and operating methods of the present invention will be apparent from the following detailed description of some embodiments thereof, made by way of a non-limiting example.

### Brief description of the figures

Reference will be made to the figures of the accompanying drawings, in which:
- figures 1 and 2 schematically show a top plan view and a lateral view, respectively, of the osteotomy device according to the present invention;
- figure 3 shows a longitudinal sectional view of the device in figure 1;
- figure 4 shows a detail of figure 1.

### Detailed description of preferred embodiments of the invention

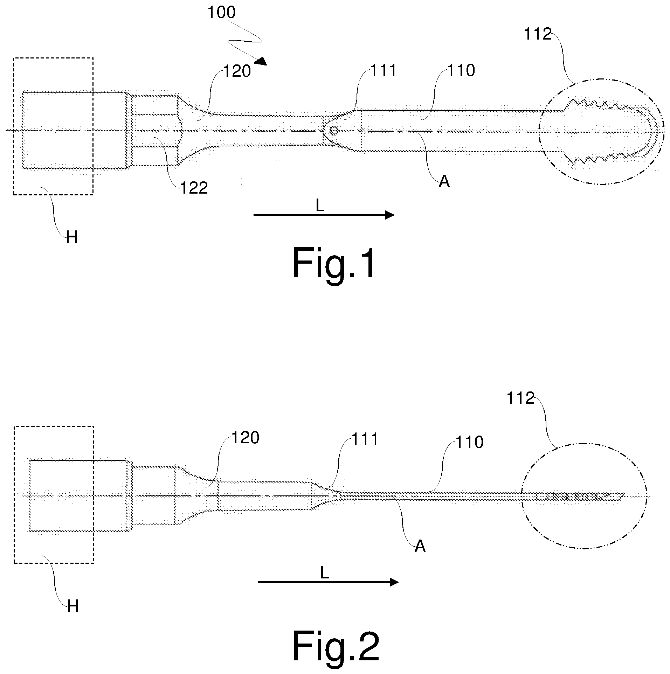

With reference initially to figures 1 to 3, an ultrasonic cutting device according to the invention is generally indicated with reference numeral 100.

The device 100 comprises a blade 110 which extends in a longitudinal direction L from an attachment member or tang 120 of the device 100 itself configured for connection to a handpiece (not shown) provided with a generator of ultrasonic vibrations directed predominantly in the longitudinal direction L. The connection between the device 100 and the handpiece is not direct, but is carried out through a special rod-like extension (not shown) suitably protected from contact with the tissues by means of a sheath.

The blade 110 has a generally flat shape and the attachment member 120, of generally cylindrical shape, is connected to the blade 110 at a proximal portion 111 thereof, of thickness gradually decreasing in the longitudinal direction L.

The blade 110 and the attachment member 120 are made in one piece and made of stainless steel for medical use, for example X40CrMoVN16-2.

The blade 110 further comprises a distal portion provided with a plurality of cutting elements, as will be described in detail hereinafter. The distal portion is indicated as a whole with reference numeral 112.

The longitudinal sides or edges of the blade 110 are generally rectilinear and respectively parallel to a longitudinal axis A of the blade 110, which is in turn parallel to the longitudinal direction L.

The connection between the device 100 and the extension member can be of threaded type and for this purpose in the connection 120, for example, a threaded hole 121 is made, for example, in the longitudinal direction. To allow the manoeuvres for screwing and unscrewing the attachment member 120 with respect to the extension member, on its outer surface there are for example at least two diametrically opposed flat surfaces 122 for manoeuvring.

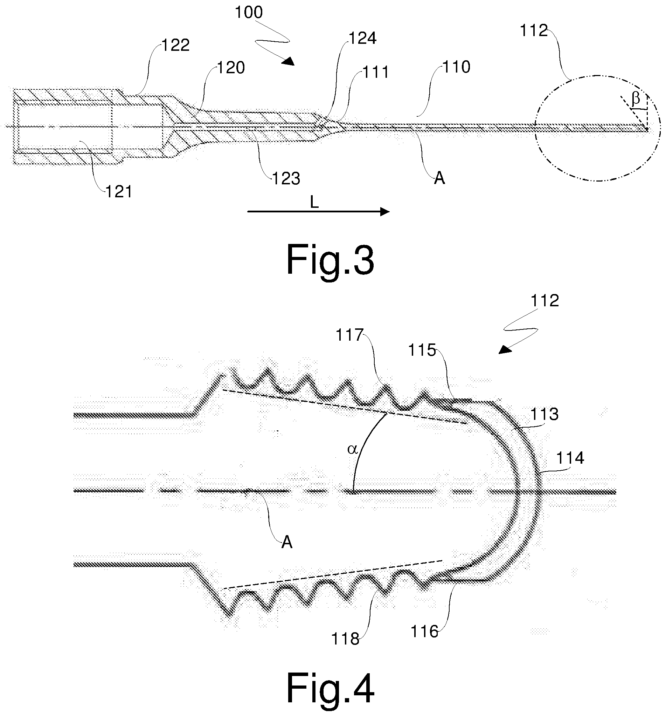

With particular reference to the longitudinal section in figure 3, the attachment member 120 further comprises a channel 123 which extends in the longitudinal direction L from the hole 121 towards the proximal portion 111 of the blade 110 and flows at an irrigation aperture 124 through which is possible to deliver a refrigerant fluid, for example a saline solution, provided by a remote supply source (not shown) connected to the handpiece.

In use, the blade 110 of the ultrasonic cutting device 100 is placed in the vicinity of the part to be cut without touching any surface. The vibration generator and the supply source of the refrigerant fluid are then started, and subsequently the blade 110 is brought into contact with the reference bone surface in the surgical site.

The blade 110 is moved alternately back and forth in the longitudinal direction L with respect to the bone tissue progressively penetrating therein. The refrigerant fluid delivered by the irrigation aperture 124 allows the removal of heat from the surgical site, favouring the penetration of the blade 110.

Referring now to figure 4, the distal portion 112 of the blade 110 comprises an arcuate portion 113 formed at the free end in the longitudinal direction L. The arcuate portion 113 develops symmetrically with respect to the longitudinal axis A of the blade 110, for example over an arc of about 180°.

The arcuate portion 113 comprises a continuous cutting profile 114 formed for example as a bevel in the thickness direction of the blade 110, as shown in the longitudinal section in figure 3. The inclination angle β of the chamfer is preferably comprised between 30° and 45°.

The continuous cutting profile 114 of the arcuate portion 113 allows obtaining a high blade guiding precision during the execution of a cut and offers a surgeon a good tactile feedback due to the continuous contact between the blade 110 and the bone tissue.

The arcuate portion 113 further comprises at its opposite ends two edges 115, 116 which join to the sides of first teeth 117, 118 of respective sets of teeth, all equal to each other, formed on both sides of the blade 110.

The teeth 117, 118 each have an isosceles triangular plan view and are preferably longitudinally spaced from each other by respective grooves which facilitate the discharge of the fragments of bone material during the cut.

The teeth 117, 118 formed on each side of the blade 110 are aligned with each other along incident directions with respect to the longitudinal axis A, schematically shown by broken lines. The inclination angle α of such incident directions is preferably comprised between 5° and 10°. The overall plan configuration of the distal portion 112 is symmetrical with respect to the longitudinal axis A and, in the section where the teeth 117, 118 are formed, progressively widens from the arcuate portion 113 towards the proximal portion 111 of the blade 110. The distal portion 112 terminates beyond the teeth 117, 118, where the blade 110 has a smaller width and a rectangular plan shape.

It will be understood that, during the execution of a cut, the conformation of the distal portion 112 causes a gradual widening of the slit created by the blade 110, which allows a surgeon to concentrate more on the cutting direction and its depth to the benefit of precision of execution of the same cuts.

Moreover, this particular configuration of the distal portion 112 contributes to the elimination of the bone material removed in synergy with a transversal component of the vibratory movement, or perpendicular to the longitudinal direction L and to the axis A of the ultrasonic device 100.

According to an embodiment of the invention (not shown), the ultrasonic cutting device 100 comprises a plurality of indentations formed in the distal portion.

The indentations, for example three, are formed in the distal portion 112 of the blade 110 in the area surrounded by the arcuate portion 113 and extend parallel to the longitudinal axis A up to the chamfer 114.

The function of the indentations is to facilitate the penetration of the device 100 into the bone tissue. More particularly, the portions of bone tissue located at the grooves of the indentations are removed from the transverse component of the vibratory motion transmitted to the ultrasonic device 100, thus facilitating penetration into the bone tissue. In other words, the indentations allow "removing" the bone material by freeing the space for the advancement of the device 100.

The present invention has been described thus far with reference to preferred embodiments thereof. It is understood that other embodiments may exist that relate to the same inventive scope, as defined by the scope of protection of the following claims.

## Claims

1. An ultrasonic cutting device (100) for osteotomy, said device comprising:
- a blade (110) extending in a longitudinal direction (L);
- an attachment member (120) configured to be connected, through a suitable extension member, with a handpiece configured to generate vibrations that are mainly directed in said longitudinal direction (L), said attachment member (120) being connected to a proximal end (111) of said blade (110);
wherein the blade (110) further comprises a plurality of cutting elements formed on a distal portion (112) thereof,
wherein said cutting elements comprise:
- an arcuate portion (113) arranged at a free end of said distal portion (112) in the longitudinal direction (L), said arcuate portion (113) having a continuous cutting edge (114);
- a plurality of teeth (117, 118) having a triangular shape, said teeth (117, 118) being formed on opposite sides of the blade (110) symmetrically with respect to a longitudinal axis (A) thereof, the teeth (117, 118) being aligned with each other along respective directions that are incident relative to said longitudinal axis (A),
the overall configuration of the distal portion (112) of the blade (110) being symmetrical relative to the longitudinal axis (A) and progressively widening from the arcuate portion (113) towards the proximal portion (111) of the blade (110) in the portion where the teeth (117, 118) are formed.

2. An ultrasonic cutting device (100) according to claim 1, wherein the arcuate portion (113) extends over an arc about 180° symmetrically with respect to the longitudinal axis (A).

3. An ultrasonic cutting device (100) according to claim 1 or 2, wherein the continuous cutting edge (114) of the arcuate portion (113) is a chamfer formed in the direction of the blade thickness (110).

4. An ultrasonic cutting device (100) according to claim 3, wherein an inclination angle (β) of said chamfer is comprised between 30° and 45°.

5. An ultrasonic cutting device (100) according to any one of claims 1 to 4, further comprising a plurality of indentations formed in the distal portion (112) of the blade (110) surrounded by the arcuate portion (113), said indentations extending parallel to the longitudinal axis (A) up to the chamfer (114).

6. An ultrasonic cutting device (100) according to any one of claims 1 to 5, wherein, according to a top plan view, each tooth (117, 118) has the shape of an isosceles triangle.

7. An ultrasonic cutting device (100) according to claim 6, wherein the teeth (117, 118) are spaced longitudinally from each other by respective grooves.

8. An ultrasonic cutting device (100) according to any one of claims 1 to 7, wherein the directions along which the teeth (117, 118) are aligned are inclined relative to the longitudinal axis (A) by an inclination angle (α) comprised between 5° and 10°.

9. An ultrasonic cutting device (100) according to any one of claims 1 to 8, wherein the attachment member (120) comprises a channel (123) for the feeding of a refrigerant fluid and an irrigation aperture (124) for dispensing said fluid, said irrigation aperture (124) being formed close to the proximal portion (111) of the blade (110).

## Patentansprüche

1. Ultraschallschneidvorrichtung (100) für Osteotomie, wobei die Vorrichtung enthält:
- eine Klinge (110), die sich in einer Längsrichtung (L) erstreckt;
- ein Befestigungselement (120), das konfiguriert ist, um durch ein geeignetes Verlängerungselement mit einem Handstück verbunden zu werden, das konfiguriert ist, um Schwingungen zu erzeugen, die im Wesentlichen in der Längsrichtung (L) gerichtet sind, wobei das Befestigungselement (120) mit einem proximalen Ende (111) der Klinge (110) verbunden ist;
wobei die Klinge (110) ferner eine Mehrzahl von Schneidelementen enthält, die an einem distalen Teil (112) davon ausgebildet sind,
wobei die Schneidelemente enthalten:
- einen bogenförmigen Teil (113), der an einem freien Ende des distalen Teils (112) in der Längsrichtung (L) angeordnet ist, wobei der bogenförmige Teil (113) eine kontinuierliche Schneidkante (114) hat;
- mehrere Zähne (117, 118), die eine dreieckige Form haben, wobei die Zähne (117, 118) auf gegenüberliegenden Seiten der Klinge (110) symmetrisch zu einer Längsachse (A) davon ausgebildet sind, wobei die Zähne (117) miteinander entlang jeweiligen Richtungen ausgerichtet sind, die relativ zu der Längsachse (A) einfallend sind,
wobei die Gesamtkonfiguration des distalen Teils (112) der Klinge (110) relativ zur Längsachse (A) symmetrisch ist und sich vom bogenförmigen Teil (113) zum proximalen Teil (111) der Klinge (110) hin in dem Teil zunehmend erweitert, in dem die Zähne (117, 118) ausgebildet sind.

2. Ultraschallschneidvorrichtung (100) nach Anspruch 1, wobei sich der bogenförmige Teil (113) über einen Bogen über 180 ° symmetrisch in Bezug auf die Längsachse (A) erstreckt.

3. Ultraschallschneidvorrichtung (100) nach Anspruch 1 oder 2, wobei die kontinuierliche Schneidkante (114) des bogenförmigen Teils (113) eine Schräge ist, die in Richtung der Klingendicke (110) ausgebildete ist.

4. Ultraschallschneidvorrichtung (100) nach Anspruch 3, wobei ein Neigungswinkel (β) der Schräge zwischen 30 ° und 45 ° enthalten ist.

5. Ultraschallschneidvorrichtung (100) nach einem der Ansprüche 1 bis 4, wobei ferner eine Mehrzahl von Vertiefungen (119) enthalten ist, die in dem distalen Teil (112) der Klinge (110) ausgebildet sind, der von dem bogenförmigen Teil (113) umgeben ist, wobei sich die Vertiefungen (119) parallel zur Längsachse (A) bis zur Schräge (114) erstrecken.

6. Ultraschallschneidvorrichtung (100) nach einem der Ansprüche 1 bis 5, wobei entsprechend einer Draufsicht jeder Zahn (117, 118) die Form eines gleichschenkligen Dreiecks hat.

7. Ultraschallschneidvorrichtung (100) nach Anspruch 6, wobei die Zähne (117, 118) durch entsprechende Rillen in Längsrichtung voneinander beabstandet sind.

8. Ultraschallschneidvorrichtung (100) nach einem der Ansprüche 1 bis 7, wobei die Richtungen, entlang denen die Zähne (117, 118) ausgerichtet sind, relativ zur Längsachse (A) um einen Neigungswinkel (α) geneigt sind, der zwischen 5 ° und 10 ° enthalten ist.

9. Ultraschallschneidvorrichtung (100) nach einem der Ansprüche 1 bis 8, wobei das Befestigungselement (120) einen Kanal (123) zum Zuführen einer Kühlmittelflüssigkeit und eine Spülöffnung (124) zum Abgeben der Flüssigkeit enthält, wobei die Spülöffnung (124) nahe dem proximalen Teil (111) der Klinge (110) ausgebildet ist.

## Revendications

1. Dispositif de coupe par ultrasons (100) pour ostéotomie, ledit dispositif comprenant :
- une lame (110) s'étendant selon une direction longitudinale (L) ;
- un élément de fixation (120) configuré pour être connecté, par l'intermédiaire d'un élément d'extension approprié, à une pièce à main configurée pour générer des vibrations principalement dirigées dans ladite direction longitudinale (L), ledit élément de fixation (120) étant connecté à l'extrémité proximale (111) de ladite lame (110) ;
dans lequel la lame (110) comprend en outre une pluralité d'éléments de coupe formés sur sa partie distale (112),
dans lequel lesdits éléments de coupe comprennent:
- une partie courbe (113) agencée sur une extrémité libre de ladite partie distale (112) dans la direction longitudinale (L), ladite partie courbe (113) ayant un bord de coupe continu (114) ;
- une pluralité de dents (117, 118) ayant une forme triangulaire, lesdites dents (117, 118) étant formées sur les côtés opposés de la lame (110) de façon symétrique par rapport à un axe longitudinal (A) de celle-ci, les dents (117, 118) étant alignées les unes avec les autres respectivement le long de directions qui coupent ledit axe longitudinal (A),
la configuration globale de la partie distale (112) de la lame (110) étant symétrique par rapport à l'axe longitudinal (A) et s'élargissant progressivement à partir de la partie courbe (113) vers la partie proximale (111) de la lame (110) dans la partie où sont formées les dents (117, 118).

2. Dispositif de coupe par ultrasons (100) selon la revendication 1, dans lequel la partie courbe (113) s'étend sur un arc d'environ 180° de façon symétrique par rapport à l'axe longitudinal (A).

3. Dispositif de coupe par ultrasons (100) selon l'une des revendications 1 et 2, dans lequel le bord de coupe continu (114) de la partie courbe (113) est un chanfrein formé selon la direction de l'épaisseur de la lame (110).

4. Dispositif de coupe par ultrasons (100) selon la revendication 3, dans lequel un angle d'inclinaison (β) dudit chanfrein est compris entre 30° et 45°.

5. Dispositif de coupe par ultrasons (100) selon l'une quelconque des revendications 1 à 4, comprenant en outre une pluralité d'indentations formées dans la partie distale (112) de la lame (110) entourée par la partie courbe (113), lesdites indentations s'étendant parallèlement à l'axe longitudinal (A) jusqu'au chanfrein (114).

6. Dispositif de coupe par ultrasons (100) selon l'une quelconque des revendications 1 à 5, dans lequel, selon une vue en plan de dessus, chaque dent (117, 118) a la forme d'un triangle isocèle.

7. Dispositif de coupe par ultrasons (100) selon la revendication 6, dans lequel les dents (117, 118) sont espacées longitudinalement les unes des autres respectivement par des rainures.

8. Dispositif de coupe par ultrasons (100) selon l'une quelconque des revendications 1 à 7, dans lequel les directions le long desquelles sont alignées les dents (117, 118) sont inclinées par rapport à l'axe longitudinal (A) selon un angle d'inclinaison (α) compris entre 5° et 10°.

9. Dispositif de coupe par ultrasons (100) selon l'une quelconque des revendications 1 à 8, dans lequel l'élément de fixation (120) comprend un canal (123) pour l'alimentation en un fluide réfrigérant et une ouverture d'irrigation (124) pour distribuer ledit fluide, ladite ouverture d'irrigation (124) étant formée à proximité de la partie proximale (111) de la lame (110).
